# EUROPEAN PATENT SPECIFICATION

(11) **EP 3 904 850 B1**
(45) Date of publication and mention of the grant of the patent: **18.09.2024**
(21) Application number: 20766584.5
(22) Date of filing: 24.01.2020
(51) Int. Cl.: G01K 1/14, A61C 19/04, A61B 5/00

(54) **INTRAORAL ORGANISM MONITORING DEVICE**
VORRICHTUNG ZUR ÜBERWACHUNG EINES INTRAORALEN ORGANISMUS
DISPOSITIF DE SURVEILLANCE D'ORGANISME INTRABUCCAL

(30) Priority: 05.03.2019 JP 2019039436
(43) Date of publication of application: 03.11.2021
(73) Proprietor: Seiko Group Corporation, Tokyo (JP); Showa University, Tokyo 142-8555 (JP)
(72) Inventor: ISOGAI, Ryosuke, Chiba-shi, Chiba 261-8507 (JP); YOSHIDA ,Yoshifumi, Chiba-shi, Chiba 261-8507 (JP); MAKI, Kotaro, Tokyo 142-8555 (JP)
(74) Representative: Lewis Silkin LLP
(86) International application number: PCT/JP2020/002562
(87) International publication number: WO 2020/179277

(56) References cited:
- WO-A1-2009/073852
- JP-A- 2013 005 995
- JP-A- H07 246 191
- US-A1- 2006 166 157
- US-A1- 2018 000 563

## Description

### Technical Field

The present invention relates to an intraoral biological monitoring device.

### Background Art

Currently, a technique to perform various treatments has been established in which a mouthpiece or the like for holding a sensor is worn in an oral cavity of a subject to acquire biological data from the subject. For example, PTL 1 discloses an intraoral-installed biological monitor including a biological sensor, a signal processing unit, a communication unit, and an operating equipment unit. The biological sensor is attached to a denture base or a mouthpiece and includes a sensor that detects a body temperature, a pulse, and enzymes or metabolites or the like in saliva, and an acceleration sensor that detects a body posture or a movement. The signal processing unit evaluates data obtained from the biological sensor, detects an abnormal value, and stores the data. The communication unit uses a wireless system to transmit the data in the signal processing unit to a management center such as a medical center, and to receive a command signal from the management center such as the medical center. The operating equipment unit is operated, based on a command signal from the management center.

### Citation List

### Patent Literature

[PTL 1] Japanese Unexamined Patent Application, First Publication No. 2004-167120

US 2018/000563 discloses intraoral appliances with sensing for detection of placement of dental aligners in a patient's mouth on teeth for indication of wearing compliance. The disclosed apparatuses and methods are for detecting wearing, including compliance, and for reliably transferring data, by wired or wireless direct or indirect communication of electronic compliance information to a smartphone. Also described are dental appliances that can detect physiological parameters related to respiration and sleep. Also described are aligner cases that enable NFC communication with electronic compliance indicator (ECI) devices and Bluetooth communication with smartphones.

The intraoral-installed biological monitor configured in this way is usually sealed in the denture base or the mouthpiece. Accordingly, it is necessary to transmit acquired data to an external device such as a computer used by a doctor with wireless communication such as Bluetooth (registered trademark) Low Energy (BLE). For example, the data referred to herein is biological data acquired while being attached to a crown of a subject, and attachment/detachment determining data which is a basis for determining whether or not the intraoral-installed biological monitor itself is installed in an oral cavity of the subject.

However, when the above-described intraoral-installed biological monitor adopts the wireless communication such as the BLE, the acquired data cannot be transmitted to the external device unless the acquired data is advertised to the external device. On the other hand, the intraoral-installed biological monitor consumes a large amount of electric power due to the advertising. Therefore, it is preferable to perform the advertising only when the acquired data is transmitted to the external device.

However, in many cases, the intraoral-installed biological monitor is miniaturized in view of a fact that the intraoral-installed biological monitor is installed to be in the intraoral of the subject. Therefore, in the intraoral-placed biological monitor, it is difficult to be switched between a case where advertising is performed and a case where advertising is not performed, and it is difficult to receive power used for the advertising from a large capacity battery.

### Summary of Invention

### Technical Problem

The present invention is made in view of the above-described problems, and an object thereof is to provide an intraoral biological monitoring device capable of performing advertising when necessary while avoiding a size increase.

### Solution to Problem

According to an aspect of the present invention, in order to achieve the above-described object, there is provided an intraoral biological monitoring device as defined in claim 1.

An intraoral biological monitoring device according to another aspect of the present invention is defined in claim 7.

### Advantageous Effects of Invention

According to the present invention, advertising can be performed when necessary while avoiding a size increase.

### Brief Description of Drawings

Fig. 1 is a view showing an example of an appearance of an intraoral biological monitoring device according to a first embodiment.
Fig. 2 is a view showing an example of a cross section in a plane passing through a sensor and a photodetector which are included in the intraoral biological monitoring device according to the first embodiment.
Fig. 3 is a view showing an example of a hardware configuration of the intraoral biological monitoring device according to the first embodiment.
Fig. 4 is a view showing an example of a functional configuration of the intraoral biological monitoring device according to the first embodiment.
Fig. 5 is a sequence diagram showing an example of a process performed by the intraoral biological monitoring device and an external device according to the first embodiment.
Fig. 6 is a view showing an example of a hardware configuration of an intraoral biological monitoring device according to a second embodiment.
Fig. 7 is a sequence diagram showing an example of a process performed by the intraoral biological monitoring device and an external device according to the second embodiment.
Fig. 8 is a view showing an example of a cross section in a plane passing through a sensor and a photodetector which are included in an intraoral biological monitoring device according to another embodiment.
Fig. 9 is a view showing an example of a cross section in a plane passing through a sensor and a photodetector which are included in an intraoral biological monitoring device according to another embodiment.
Fig. 10 is a view showing an example of a cross section in a plane passing through a sensor and a photodetector which are included in an intraoral biological monitoring device according to another embodiment.

### Description of Embodiments

### [First Embodiment]

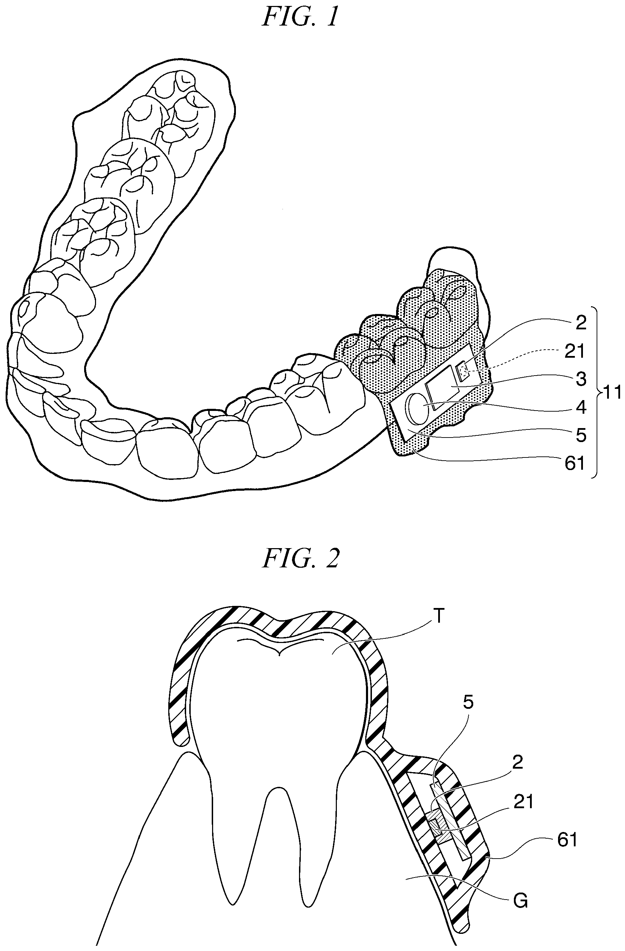

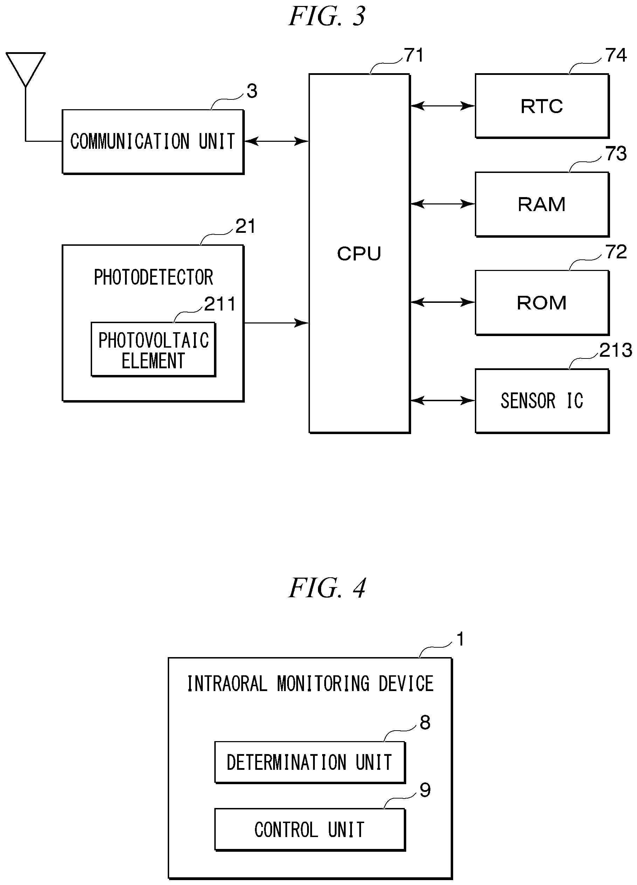

An example of an intraoral biological monitoring device according to a first embodiment will be described with reference to Figs. 1 to 4. Fig. 1 is a view showing an example of an appearance of the intraoral biological monitoring device according to the first embodiment. Fig. 2 is a view showing an example of a cross section in a plane passing through a sensor and a photodetector which are included in the intraoral biological monitoring device according to the first embodiment. Fig. 3 is a view showing an example of a hardware configuration of the intraoral biological monitoring device according to the first embodiment.

As shown in Figs. 1, 2, and 3, an intraoral biological monitoring device 11 includes a sensor 2, a communication unit 3, a battery 4, a substrate 5, a mouthpiece 61, a central processing unit (CPU) 71, a read only memory (ROM) 72, a random access memory (RAM) 73, and a real-time clock (RTC) 74.

The sensor 2 is an optical sensor including a photodetector 21 that detects light, and is disposed in a gum G of a subject. The sensor 2 is disposed in the gum G of the subject. For example, the photodetector 21 detects the light having a wavelength belonging to a near infrared region or a visible light region. The light detected by the photodetector 21 may be subjected to a predetermined modulation. The predetermined modulation referred to herein means optionally changing a wavelength, an intensity, a timing at which an output starts, or a timing at which the output ends. Furthermore, the photodetector 21 may include a photovoltaic element 211 that receives the light to generate electric power, and may detect the light by using the electric power generated by the photovoltaic element 211. The sensor 2 may be controlled by a sensor integrated circuit (IC) 213 shown in Fig. 3, and may be intermittently operated, for example, every 10 seconds.

The communication unit 3 performs communication, that is, advertising, in response to an operation in which a signal is input to the sensor 2. For example, the communication unit 3 performs the communication in response to an operation in which the photodetector 21 detects the light. The length of one advertising is 20 seconds, for example. The communication unit 3 performs the communication with an external device 100 (to be described later) in order to transmit attachment/detachment determining data acquired by the intraoral biological monitoring device 11 to the external device 100. The battery 4 supplies the electric power to each unit of the intraoral biological monitoring device 11. The substrate 5 holds the sensor 2, the communication unit 3, and the battery 4.

The mouthpiece 61 holds the sensor 2, the communication unit 3, the battery 4, and the substrate 5, is made of a light-transmitting material, and is attached to a crown T of the subject in a state where the photodetector 21 faces at least one of the crown T and the gum G of the subject. Light transmittance of this material is preferably 20% or higher, for example. The mouthpiece 61 covers at least a part of the gum G of the subject. Furthermore, the mouthpiece 61 covers at least the photodetector 21 in the sensor 2. Specifically, the mouthpiece 61 seals the sensor 2 together with the communication unit 3, the battery 4, and the substrate 5.

Fig. 4 is a view showing an example of a functional configuration of the intraoral biological monitoring device according to the first embodiment. The CPU 71 reads various programs stored in the read only memory (ROM) 72 when necessary, deploys various programs in the random access memory (RAM) 73, and executes various programs to function as the determination unit 8 and the control unit 9 which are shown in Fig. 4. The RTC 74 activates the CPU 71 which is normally in a sleep state, when necessary.

The determination unit 8 determines that the mouthpiece 61 is attached to the crown T of the subject, when the sensor 2 does not detect the light, and determines that the mouthpiece 61 is not attached to the crown T of the subject, when the sensor 2 detects the light. The control unit 9 controls an operation of each unit of the intraoral biological monitoring device 11, for example, the sensor 2 and the communication unit 3, when necessary.

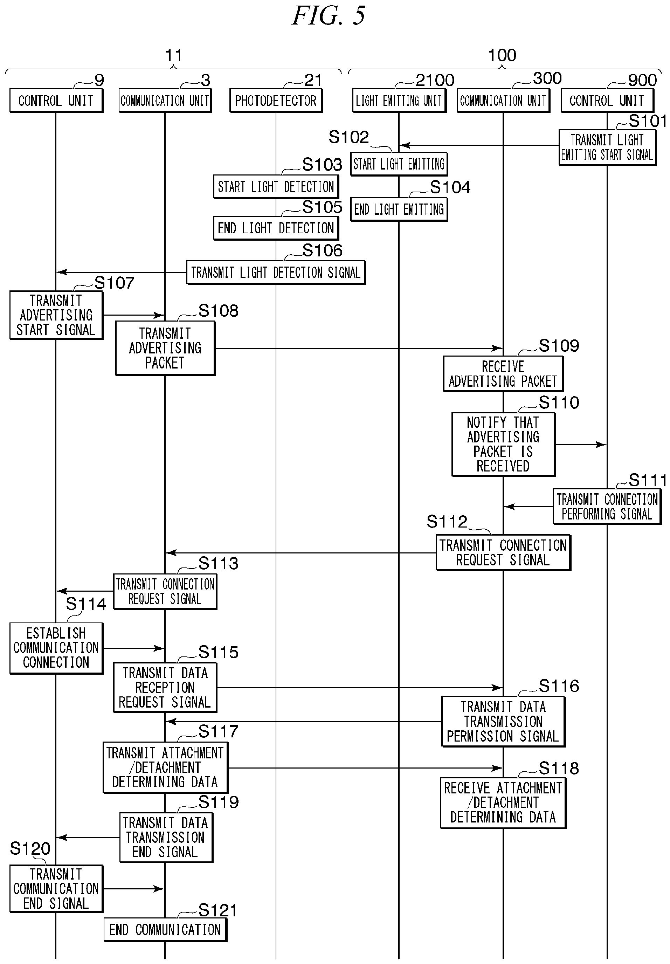

Next, an example of an operation of the intraoral biological monitoring device 11 according to the first embodiment will be described with reference to Fig. 5. Fig. 5 is a sequence diagram showing an example of a process performed by the intraoral biological monitoring device and the external device according to the first embodiment.

Fig. 5 shows an example of a process performed when the intraoral biological monitoring device 11 transmits the attachment/detachment determining data acquired by an attachment/detachment determining sensor (not shown) to the external device 100. The attachment/detachment determining sensor is an optical sensor or a temperature sensor, for example.

For example, when the attachment/detachment determining sensor is an optical sensor, the period during which the optical sensor does not detect light corresponds to the period during which the intraoral biological monitoring device 11 is attached to the crown T of the subject. On the other hand, a period during which the optical sensor detects the light corresponds to a period during which the intraoral biological monitoring device 11 is not attached to the crown T of the subject.

Alternatively, when the attachment/detachment determining sensor is the temperature sensor, a period during which a temperature detected by the temperature sensor is approximately the same as a body temperature of the subject corresponds to a period during which the intraoral biological monitoring device 11 is attached to the crown T of the subject. On the other hand, a period during which the temperature detected by the temperature sensor is different from the body temperature of the subject by a prescribed temperature or higher corresponds to a period during which the intraoral biological monitoring device 11 is not attached to the crown T of the subject.

For example, the external device 100 is a computer used by a doctor. Based on data acquireed by the attachment/detachment determining sensor, the external device 100 estimates the period during which the intraoral biological monitoring device 11 is attached to the crown T of the subject and the period during which the intraoral biological monitoring device 11 is not attached to the crown T of the subject. The external device 100 includes a communication unit 300, a control unit 900, and a light emitting unit 2100.

The communication unit 300 performs the communication with the intraoral biological monitoring device 11. The control unit 900 controls an operation of each unit of the external device 100, when necessary. Under the control of the control unit 900, the light emitting unit 2100 outputs the light subjected to the above-described predetermined modulation, to the photodetector 21.

In Step S101, the control unit 900 transmits a light emitting start signal instructing the light emitting unit 2100 to start light emitting to the light emitting unit 2100. For example, the control unit 900 transmits the light emitting start signal to the light emitting unit 2100, when a switch provided in the external device 100 is operated or a request is made from a program installed in the external device 100.

In Step S102, the light emitting unit 2100 starts light emitting.

In Step S103, the photodetector 21 starts light detection.

In Step S104, the light emitting unit 2100 ends the light emitting. A time from a start to an end of the light emitting of the light emitting unit 2100 is set in advance.

In Step S105, the photodetector 21 ends the light detection.

In Step S106, the photodetector 21 transmits a light detection signal indicating that the light is detected, to the control unit 9.

In Step S107, the control unit 9 transmits an advertising start signal instructing the communication unit 3 to start advertising, to the communication unit 3.

In Step S108, the communication unit 3 transmits an advertising packet to the communication unit 300.

In Step S109, the communication unit 300 receives the advertising packet from the communication unit 3.

In Step S110, the communication unit 300 notifies the control unit 900 that the advertising packet is received.

In Step S111, the control unit 900 transmits a connection performing signal instructing to the communication unit 3 to transmit a connection request signal requesting the intraoral biological monitoring device 11 for communication connection, to the communication unit 3.

In Step S112, the communication unit 300 transmits the connection request signal to the communication unit 3.

In Step S113, the communication unit 3 transfers the connection request signal received from the communication unit 300 to the control unit 9.

In Step S114, the control unit 9 causes the communication unit 3 to establish the communication connection with the external device 100.

In Step S115, the communication unit 3 transmits a data reception request signal requesting the external device 100 to receive attachment/detachment determining data to the communication unit 300.

In Step S116, the communication unit 300 transmits a data transmission permission signal permitting the transmission of attachment/detachment determining data to the intraoral biological monitoring device 11.

In Step S 117, the communication unit 3 transmits the attachment/detachment determining data to the communication unit 300.

In Step S118, the communication unit 300 receives the attachment/detachment determining data from the communication unit 3.

In Step S119, the communication unit 3 transmits a data transmission end signal indicating that the attachment/detachment determining data is completely transmitted to the external device 100, to the control unit 9.

In Step S120, the control unit 9 transmits a communication end signal instructing the communication unit 3 to end the communication with the external device 100.

In Step S121, the communication unit 3 ends the communication with the external device 100.

Hitherto, the intraoral biological monitoring device 11 according to the first embodiment has been described. The intraoral biological monitoring device 11 performs the communication with the external device 100 in response to an operation in which a signal is input to the sensor 2. Therefore, the intraoral biological monitoring device 11 does not require a switch for switching between a case where the advertising is performed and a case where the advertising is not performed. Accordingly, a size increase can be avoided. In addition, the intraoral biological monitoring device 11 can perform the advertising only when necessary. Furthermore, the intraoral biological monitoring device 11 can avoid the electric power stored in the battery 4 from being wasted by performing the advertising more than necessary. Accordingly, the size increase caused by an increase in size of the battery 4 can be avoided.

In addition, as the sensor 2, the intraoral biological monitoring device 11 adopts the optical sensor including the photodetector 21 made of a light-transmitting material and covered with the mouthpiece 61. The sensor 2 is smaller than the switch for switching between a case where the advertising is performed and a case where the advertising is not performed. Therefore, the intraoral biological monitoring device 11 can perform the advertising simply by inputting the light to the photodetector 21 while avoiding the size increase.

In addition, the mouthpiece 61 is attached to the crown T of the subject in a state where the photodetector 21 faces at least one of the crown T and the gum G of the subject. In this manner, the intraoral biological monitoring device 11 is attached to the crown T of the subject. It is possible to avoid a situation in which the advertising is unnecessarily performed since the light is input to the photodetector 21 when the subject opens his or her mouth. Simultaneously, the intraoral biological monitoring device 11 can perform the advertising by inputting the light to the photodetector 21 only when the intraoral biological monitoring device 11 performs the communication with the external device 100 after being detached from the crown T of the subject.

In addition, the photodetector 21 detects the light having a wavelength belonging to the near infrared region of the visible light region. In this manner, the intraoral biological monitoring device 11 avoids a situation in which an oral cavity of the subject is irradiated with ultraviolet rays that may adversely affect a human body, and dental materials used for treatment of the oral cavity of the subject can be more widely selected.

In addition, the photodetector 21 detects the light subjected to a predetermined modulation. In this manner, the intraoral biological monitoring device 11 can avoid a situation in which the advertising is unnecessarily performed due to disturbance of the light input to the photodetector 21 when the subject opens his or her mouth.

In addition, the photodetector 21 includes a photovoltaic element that receives the light to generate electric power, and detects the light by using the electric power generated by the photovoltaic element. In this manner, the intraoral biological monitoring device 11 can less rely on the battery 4 for the electric power required for the advertising, and can save the electric power stored in the battery 4.

In addition, the sensor 2 is intermittently operated, for example, every 10 seconds. In this manner, the intraoral biological monitoring device 11 can reduce the electric power consumed by the sensor 2, and can save the electric power stored in the battery 4.

In addition, the intraoral biological monitoring device 11 includes the determination unit 8 for determining that the mouthpiece 61 is attached to the crown T of the subject, when the sensor 2 does not detect the light, and determining that the mouthpiece 61 is not attached to the crown T of the subject, when the sensor 2 detects the light. In this manner, the intraoral biological monitoring device 11 can omit the attachment/detachment determining sensor for acquiring the attachment/detachment determining data, and can avoid a size increase. Alternatively, based on the attachment/detachment determining data acquired from both the attachment/detachment determining sensor and the sensor 2, the intraoral biological monitoring device 11 can complexly and accurately determine whether or not the intraoral biological monitoring device 11 itself is attached to the crown T of the subject.

### [Second Embodiment]

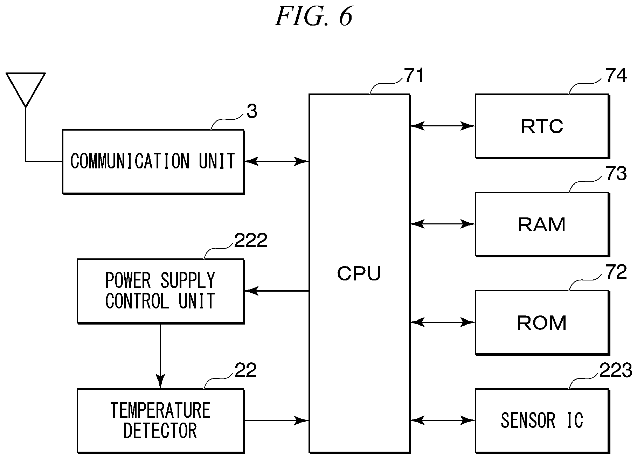

An example of an intraoral biological monitoring device according to a second embodiment will be described with reference to Fig. 6. Fig. 6 is a view showing an example of a hardware configuration of the intraoral biological monitoring device according to the second embodiment. An intraoral biological monitoring device 12 according to the second embodiment includes a sensor including a temperature detector 22 instead of the sensor 2 including the photodetector 21 included in the intraoral biological monitoring device 11 according to the first embodiment. In the following description, with regard to the intraoral biological monitoring device 12 according to the second embodiment, points different from those of the intraoral biological monitoring device 11 according to the first embodiment will be mainly described, and repeated description of the first embodiment will be omitted as appropriate.

The intraoral biological monitoring device 12 includes the sensor 2, the communication unit 3, the battery 4, the substrate 5, the mouthpiece 61, the determination unit 8, and the control unit 9 which are described above. In addition, the intraoral biological monitoring device 12 includes the CPU 71, the ROM 72, the RAM 73, and the RTC 74 which are shown in Fig. 6.

The sensor 2 included in the intraoral biological monitoring device 12 is a temperature sensor including the temperature detector 22 that detects a temperature. The communication unit 3 performs the communication in response to an operation in which the temperature detector 22 detects a temperature rise equal to or greater than a predetermined first threshold value. For example, the predetermined threshold value is a temperature of 150 degrees Celsius or higher, and 60 degrees Celsius. The temperature of 150 degrees Celsius or higher referred to herein is an example of a temperature at which the temperature detector 22 fails due to a high temperature. The temperature of 60 degrees Celsius referred to herein is an example of a temperature which is sufficiently higher than the body temperature of the subject and is relatively easily raised.

The temperature detector 22 is covered with the mouthpiece 61 made of a light-transmitting material. The communication unit 3 performs the communication in response to an operation in which the temperature detector 22 detects a temperature rise equal to or greater than the predetermined first threshold value when the temperature detector 22 is irradiated with the light. The sensor 2 may be controlled by a sensor IC 223 shown in Fig. 6, and may be intermittently operated. Furthermore, the electric power supplied to the temperature detector 22 may be controlled by a power supply control unit 222 shown in Fig. 6.

The determination unit 8 determines that the mouthpiece 61 is attached to the crown T of the subject, when the sensor 2 detects a temperature equal to or greater than a predetermined second threshold value, and determines that the mouthpiece 61 is not attached to the crown T of the subject, when the sensor 2 detects a temperature smaller than the predetermined second threshold value.

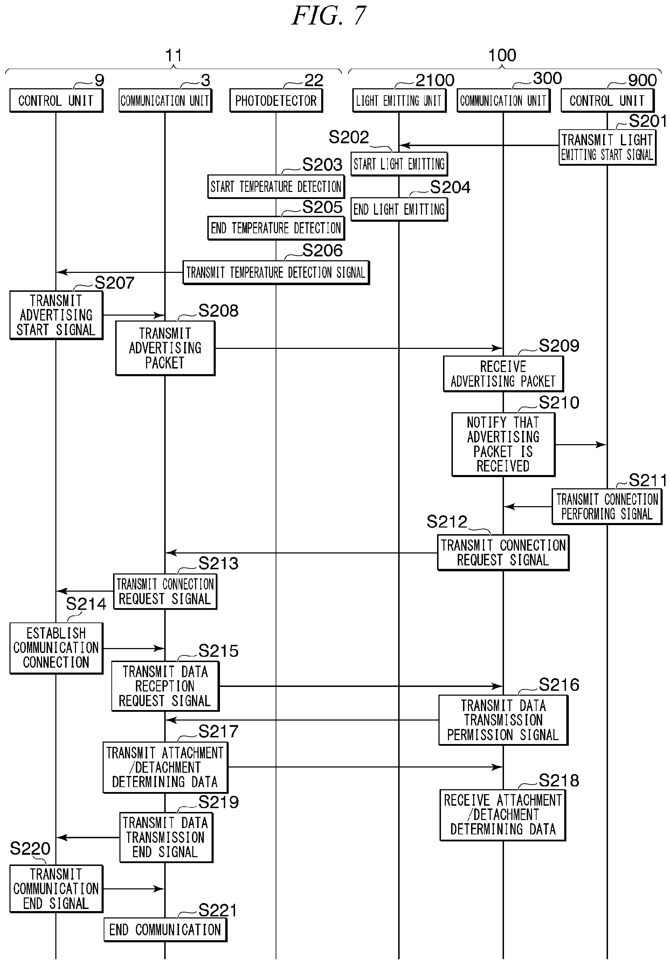

Next, an example of an operation of the intraoral biological monitoring device 12 according to the second embodiment will be described with reference to Fig. 7. Fig. 7 is a sequence diagram showing an example of a process performed by the intraoral biological monitoring device and the external device according to the second embodiment. Step 201 and Step S202, Step S204, and Step S207 to Step S221 which are shown in Fig. 7 are the same as Step 101 and Step S102, Step S104, and Step S107 to Step S121 which are shown in Fig. 5. Therefore, in the following description, Step S203, Step S205, and Step S206 will be described.

In Step S204, the temperature detector 22 starts temperature detection.

In Step S206, the temperature detector 22 ends the temperature detection.

In Step S207, the temperature detector 22 transmits a temperature detection signal indicating that the temperature is detected, to the control unit 9.

Hitherto, the intraoral biological monitoring device 12 according to the second embodiment has been described. The intraoral biological monitoring device 12 performs the communication with the external device 100 in response to an operation in which a signal is input to the sensor 2. Therefore, the intraoral biological monitoring device 12 does not require a switch for switching between a case where the advertising is performed and a case where the advertising is not performed. Accordingly, a size increase can be avoided. In addition, the intraoral biological monitoring device 12 can perform the advertising only when necessary. Furthermore, the intraoral biological monitoring device 12 can avoid the electric power stored in the battery 4 from being wasted by performing the advertising more than necessary. Accordingly, the size increase caused by an increase in size of the battery 4 can be avoided.

In addition, as the sensor 2, the intraoral biological monitoring device 12 adopts the temperature sensor including the temperature detector 22, and performs the communication in response to an operation in which the temperature detector 22 detects a temperature rise equal to or greater than the predetermined first threshold value. The sensor 2 is smaller than the switch for switching between a case where the advertising is performed and a case where the advertising is not performed. Therefore, the intraoral biological monitoring device 12 can perform the advertising simply by inputting the light or heat to the temperature detector 22 and increasing the temperature while avoiding the size increase.

In addition, the communication unit 3 adopts the temperature of 60 degrees Celsius, as the predetermined first threshold value. In this manner, the intraoral biological monitoring device 12 can avoid a situation in which the communication unit 3 unnecessarily performs the advertising in response to an operation in which the temperature detector 22 detects the temperature rise caused by the body temperature of the subject.

In addition, the communication unit 3 adopts the temperature of 150 degrees Celsius or higher, as the predetermined first threshold value. In this manner, the intraoral biological monitoring device 12 can avoid a situation in which the communication unit 3 unnecessarily performs the advertising in response to an operation in which the temperature detector 22 detects the temperature rise caused by the body temperature of the subject. In addition, when the mouthpiece 61 is used for orthodontics, in many cases, it is premised as follows in the intraoral biological monitoring device 12. The attachment/detachment determining data is discarded as it is after the attachment/detachment determining data is acquired for a certain period of time. In this case, there is no particular problem even when the temperature detector 22 fails due to a high temperature.

In addition, the intraoral biological monitoring device 12 performs the communication in response to an operation in which the temperature detector 22 detects a temperature rise equal to or greater than the predetermined first threshold value when the temperature detector 22 covered with the mouthpiece 61 made of a light-transmitting material is irradiated with the light. Therefore, the intraoral biological monitoring device 12 can avoid a situation in which a part other than the temperature detector 22 is damaged due to the heat by using the light to locally heat the temperature detector 22.

In addition, the sensor 2 is intermittently operated. In this manner, the intraoral biological monitoring device 12 can reduce the electric power consumed by the sensor 2, and can save the electric power stored in the battery 4.

In addition, the intraoral biological monitoring device 12 includes the determination unit 8 for determining that the mouthpiece is attached to the crown T of the subject, when the sensor 2 detects a temperature equal to or greater than the predetermined second threshold value, and determining that the mouthpiece 61 is not attached to the crown T of the subject, when the sensor 2 detects a temperature smaller than the predetermined second threshold value. In this manner, the intraoral biological monitoring device 12 can omit the attachment/detachment determining sensor for acquiring the attachment/detachment determining data, and can avoid a size increase. Alternatively, based on the attachment/detachment determining data acquired from both the attachment/detachment determining sensor and the sensor 2, the intraoral biological monitoring device 11 can complexly and accurately determine whether or not the intraoral biological monitoring device 11 itself is attached to the crown T of the subject.

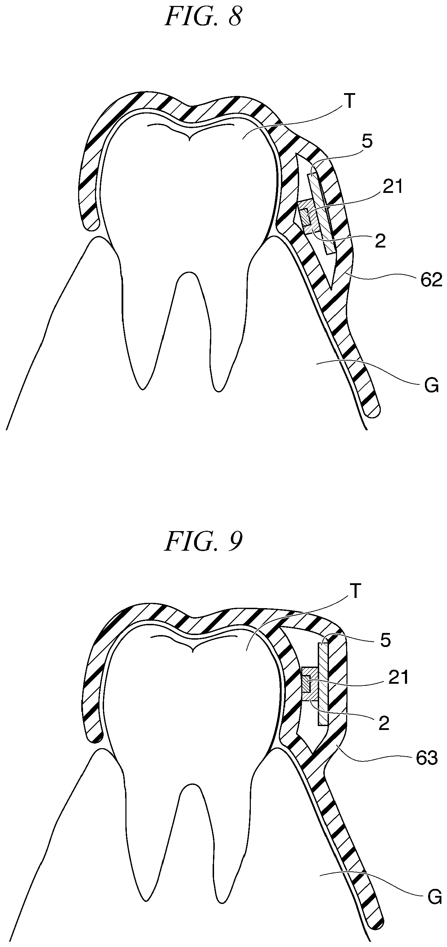

In the first embodiment and the second embodiment which are described above, as shown in Fig. 2, a case where the sensor 2 is disposed in the gum G of the subject has been described as an example. However, the present invention is not limited thereto. Fig. 8 is a view showing an example of a cross section in a plane passing through a sensor and a photodetector which are included in an intraoral biological monitoring device according to another embodiment. As shown in Fig. 8, the sensor 2 may be disposed in the crown T and the gum G of the subject. In this case, the intraoral biological monitoring device 11 according to the first embodiment and the intraoral biological monitoring device 12 according to the second embodiment include a mouthpiece 62 shown in Fig. 8 instead of the mouthpiece 61 described above.

Fig. 9 is a view showing an example of a cross section in a plane passing through a sensor and a photodetector which are included in an intraoral biological monitoring device according to another embodiment. As shown in Fig. 9, the sensor 2 may be disposed in the crown T of the subject. In this case, the intraoral biological monitoring device 11 and the intraoral biological monitoring device 12 include a mouthpiece 63 shown in Fig. 9 instead of the mouthpiece 61 described above.

That is, the sensor 2 is disposed in at least one of a part of the crown T of the subject which does not come into contact with other teeth and the gum G of the subject, even when the subject occludes a tooth of an upper jaw and a tooth of a lower jaw. In this manner, the intraoral biological monitoring device 11 according to the first embodiment and the intraoral biological monitoring device 12 according to the second embodiment can avoid a situation in which occlusion of the subject is affected.

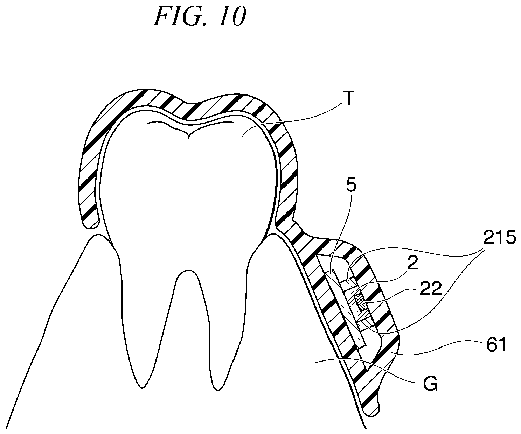

In addition, in the first embodiment described above, as shown in Fig. 2, a case where the mouthpiece 61 is attached to the crown T of the subject in a state where the photodetector 21 faces at least one of the crown T and the gum G of the subject has been described as an example. However, the present invention is not limited thereto. Fig. 10 is a view showing an example of a cross section in a plane passing through a sensor and a photodetector which are included in an intraoral biological monitoring device according to another embodiment. As shown in Fig. 10, the mouthpiece 61 may be attached to the crown T of the subject in a state where the sensor 2 faces a direction different from a direction in which the crown T and the gum G of the subject are present. When the sensor 2 includes the photodetector 21 or the temperature detector 22, the photodetector 21 or the temperature detector 22 may be in a state of facing a direction different from the direction in which the crown T and the gum G of the subject are present. In this manner, the intraoral biological monitoring device 11 or the intraoral biological monitoring device 12 can perform the advertising in response to an operation in which the light or the temperature rise is detected, even when the mouthpiece 61 is attached to the crown T of the subject.

In addition, when the sensor 2 includes the temperature detector 22, as shown in Fig. 10, the intraoral biological monitoring device 12 may include a light-absorbent member 215 provided in a region irradiated with the light to absorb the light. For example, the light-absorbent member 215 is made of a material containing carbon, and is attached to the substrate 5 to surround the temperature detector 22. In this manner, the intraoral biological monitoring device 12 can efficiently convert light irradiation into heat, and can perform the communication in response to an operation in which a temperature rise equal to or greater than the predetermined first threshold value is detected.

In addition, in the first embodiment and the second embodiment, a case where the sensor 2 is sealed in the mouthpiece 61 has been described as an example. However, the present invention is not limited thereto. For example, the mouthpiece 61 may only cover a part of the sensor 2. Alternatively, the mouthpiece 61 may cover a part except for a part where the photodetector 21 or the temperature detector 22 fetches the light.

In addition, in the first embodiment and the second embodiment, a case where the whole mouthpiece 61 is made of the light-transmitting material has been described as an example. However, the present invention is not limited thereto. For example, the mouthpiece 61 may be made of a material which does not transmit the light, except for a part where the photodetector 21 or the temperature detector 22 fetches the light. In addition, when the sensor 2 includes the temperature detector 22 and the temperature detector 22 detects the temperature rise caused by heating instead of the temperature rise caused by light irradiation, the whole mouthpiece 61 may be made of a material which does not transmit the light.

In addition, in the first embodiment and the second embodiment, a case where the intraoral biological monitoring device 11 and the intraoral biological monitoring device 12 include the CPU 71, the ROM 72, and the RAM 73 has been described as an example. However, the present invention is not limited thereto. For example, the intraoral biological monitoring device 11 and the intraoral biological monitoring device 12 may include a microcomputer having the three functions instead of the CPU 71, the ROM 72, and the RAM 73.

Similarly, in the first embodiment and the second embodiment, a case where the intraoral biological monitoring device 11 and the intraoral biological monitoring device 12 include the CPU 71, the ROM 72, the RAM 73, the RTC 74, and the communication unit 3 has been described as an example. However, the present invention is not limited thereto. For example, the intraoral biological monitoring device 11 and the intraoral biological monitoring device 12 may include a microcomputer having the five functions instead of the CPU 71, the ROM 72, the RAM 73, the RTC 74, and the communication unit 3.

In addition, in the first embodiment and the second embodiment, a case where the external device 100 irradiates the temperature detector 22 with the light by using the light emitting unit 2100 has been described as an example. However, the present invention is not limited thereto. For example, the external device 100 may include a heat generating unit having a heat source such as a heating wire instead of the light emitting unit 2100, and the temperature detector 22 may be heated by using the heat generating unit.

In addition, the functions included in the above-described intraoral biological monitoring device 11 may be entirely or partially recorded as a program on a computer-readable recording medium, and the program may be executed by a computer system. The computer system includes hardware such as a peripheral device. In addition, for example, the computer-readable recording medium includes a portable medium such as a flexible disk, a magneto-optical disk, a ROM, and a CD-ROM, a storage device such as a hard disk incorporated in the computer system, or a RAM included in a server on a network such as the Internet. A volatile memory is an example of a recording medium that holds a program for a certain period of time.

In addition, the above-described program may be transmitted to another computer system by a transmission medium, for example, a network such as the Internet or a communication line such as a telephone line.

In addition, the above-described program may be a program that entirely or partially realizes the above-described functions. The program that partially realizes the above-described functions may be a program that can realize the above-described functions in combination with a program recorded in advance in the computer system, that is, a so-called difference program.

Hitherto, the embodiments of the present invention have been described with reference to the drawings. However, a specific configuration is not limited to the above-described embodiments, and includes a design change within the scope not departing from the concept of the present invention.

### Reference Signs List

11, 12: Intraoral biological monitoring device
2: Sensor
21: Photodetector
22: Temperature detector
211: Photovoltaic element
213: Sensor IC
215: Light-absorbent member
222: Power supply control unit
223: Sensor IC
3: Communication unit
4: Battery
5: Substrate
61, 62, 63: Mouthpiece
71: CPU
72: RAM
73: ROM
74: RTC
8: Determination unit
9: Control unit
100: External device
300: Communication unit
800: Control unit
2100: Light emitting unit
G: Gum
T: Crown

## Claims

1. An intraoral biological monitoring device (11) comprising:
a sensor (2);
a communication unit (3) that is configured to perform communication in response to an operation in which a signal is input to the sensor (2);
a mouthpiece (61, 62, 63) that holds the sensor (2) and the communication unit (3), and is configured to be attached to a crown (T) of a subject, and covers at least a part of a gum (G) of the subject; and
a determination unit (8),
wherein the sensor (2) is an optical sensor including a photodetector (21) that is configured to detect light,
the mouthpiece (61, 62, 63) is made of a light-transmitting material, and covers at least the photodetector (21) of the sensor (2), and
the communication unit (3) is configured to perform the communication in response to an operation in which the light is detected by the photodetector (21),
**characterized in that**
the photodetector (21) is configured to face at least one of the crown (T) and the gum (G) of the subject by attachment of the mouthpiece (61, 62, 63) to the crown (T) of the subject, and
the determination unit (8) is configured to determine that the mouthpiece (61, 62, 63) is attached to the crown (T) of the subject when the sensor (2) does not detect the light, and to determine that the mouthpiece (61, 62, 63) is not attached to the crown (T) of the subject when the sensor (2) detects the light.

2. The intraoral biological monitoring device (11) according to claim 1,
wherein the sensor (2) is disposed in at least one of a part of the crown (T) of the subject which does not come into contact with other teeth, even when the subject occludes a tooth of an upper jaw and a tooth of a lower jaw, and the gum (G) of the subject.

3. The intraoral biological monitoring device (11) according to claim 1 or 2,
wherein the photodetector (21) is configured to detect the light having a wavelength belonging to a near infrared region or a visible light region.

4. The intraoral biological monitoring device (11) according to any one of claims 1 to 3,
wherein the photodetector (21) is configured to detect the light subjected to predetermined modulation.

5. The intraoral biological monitoring device (11) according to claim 1,
wherein the photodetector (21) includes a photovoltaic element (211) that is configured to receive the light to generate electric power, and to detect the light by using the electric power generated by the photovoltaic element (211).

6. The intraoral biological monitoring device (11) according to any one of claims 1 to 5,
wherein the sensor (2) is intermittently operated.

7. An intraoral biological monitoring device (11) comprising:
a sensor (2);
a communication unit (3) that is configured to perform communication in response to an operation in which a signal is input to the sensor (2);
a mouthpiece (61, 62, 63) that holds the sensor (2) and the communication unit (3), and is configured to be attached to a crown (T) of a subject, and covers at least a part of a gum (G) of the subject;
a light-absorbent member (215), and
a determination unit (8),
wherein the sensor (2) is a temperature sensor including a temperature detector (22) that is configured to detect a temperature,
the communication unit (3) is configured to perform the communication in response to an operation in which the temperature detector (22) detects a temperature rise equal to or greater than a predetermined first threshold value,
wherein the mouthpiece (61, 62, 63) is made of a light-transmitting material, and covers at least the temperature detector (22) in the sensor (2), and
wherein the determination unit (8) is configured to determine that the mouthpiece (61, 62, 63) is attached to the crown (T) of the subject when the sensor (2) detects a temperature equal to or greater than a predetermined second threshold value, and to determine that the mouthpiece (61, 62, 63) is not attached to the crown (T) of the subject when the sensor (2) detects a temperature smaller than the predetermined second threshold value,
**characterized in that**
the light-absorbent member (215) is provided in a region irradiated with light to absorb the light,
wherein the communication unit (3) is configured to perform the communication in response to an operation in which the temperature detector (22) detects a temperature rise equal to or greater than the predetermined first threshold value when the temperature detector (22) is irradiated with the light.

8. The intraoral biological monitoring device (11) according to claim 7,
wherein the communication unit (3) is configured to adopt a temperature of 60 degrees Celsius, as the predetermined first threshold value.

9. The intraoral biological monitoring device (11) according to claim 7,
wherein the communication unit (3) is configured to adopt a temperature of 150 degrees Celsius or higher, as the predetermined first threshold value.

10. The intraoral biological monitoring device (11) according to any one of claims 7 to 9, wherein the sensor (2) is intermittently operated.

## Patentansprüche

1. Intraorale biologische Überwachungseinrichtung (11), die Folgendes umfasst:
einen Sensor (2),
eine Kommunikationseinheit (3), die dafür konfiguriert ist, als Reaktion auf eine Operation, bei der ein Signal in den Sensor (2) eingegeben wird, eine Kommunikation durchzuführen,
ein Mundstück (61, 62, 63), das den Sensor (2) und die Kommunikationseinheit (3) hält und dafür konfiguriert ist, an einer Zahnkrone (T) eines Subjekts befestigt zu werden, und zumindest einen Teil eines Zahnfleischs (G) des Subjekts bedeckt, und
eine Feststellungseinheit (8),
wobei der Sensor (2) ein optischer Sensor ist, der einen Photodetektor (21) einschließt, der dafür konfiguriert ist, Licht zu erfassen,
das Mundstück (61, 62, 63) aus einem lichtdurchlässigen Material hergestellt ist und zumindest den Photodetektor (21) des Sensors (2) bedeckt und
die Kommunikationseinheit (3) dafür konfiguriert ist, die Kommunikation als Reaktion auf eine Operation durchzuführen, bei der das Licht durch den Photodetektor (21) erfasst wird,
**dadurch gekennzeichnet, dass**
der Photodetektor (21) dafür konfiguriert ist, durch Befestigung des Mundstücks (61, 62, 63) an der Zahnkrone (T) des Subjekts mindestens einem von der Zahnkrone (T) und dem Zahnfleisch (G) des Subjekts gegenüberzuliegen, und
die Feststellungseinheit (8) dafür konfiguriert ist, festzustellen, dass das Mundstück (61, 62, 63) an der Zahnkrone (T) des Subjekts befestigt ist, wenn der Sensor (2) das Licht nicht erfasst, und festzustellen, dass das Mundstück (61, 62, 63) nicht an der Zahnkrone (T) des Subjekts befestigt ist, wenn der Sensor (2) das Licht erfasst.

2. Intraorale biologische Überwachungseinrichtung (11) nach Anspruch 1,
wobei der Sensor (2) in mindestens einem von einem Teil der Zahnkrone (T) des Subjekts, der nicht in Berührung mit anderen Zähnen kommt, selbst, wenn das Subjekt einen Zahn eines Oberkiefers und einen Zahn eines Unterkiefers okkludiert, und dem Zahnfleisch (G) des Subjekts angeordnet ist.

3. Intraorale biologische Überwachungseinrichtung (11) nach Anspruch 1 oder 2,
wobei der Photodetektor (21) dafür konfiguriert ist, das Licht zu erfassen, das eine Wellenlänge aufweist, die zu einem Bereich nahe Infrarot oder einem Bereich sichtbaren Lichts gehört.

4. Intraorale biologische Überwachungseinrichtung (11) nach einem der Ansprüche 1 bis 3,
wobei der Photodetektor (21) dafür konfiguriert ist, das Licht zu erfassen, das einer vorbestimmten Modulation unterworfen ist.

5. Intraorale biologische Überwachungseinrichtung (11) nach Anspruch 1,
wobei der Photodetektor (21) ein photovoltaisches Element (211) einschließt, das dafür konfiguriert ist, das Licht aufzunehmen, um elektrische Energie zu erzeugen, und das Licht durch Verwenden der elektrischen Energie, die durch das photovoltaische Element (211) erzeugt wird, zu erfassen.

6. Intraorale biologische Überwachungseinrichtung (11) nach einem der Ansprüche 1 bis 5,
wobei der Sensor (2) intermittierend betrieben wird.

7. Intraorale biologische Überwachungseinrichtung (11), die Folgendes umfasst:
einen Sensor (2),
eine Kommunikationseinheit (3), die dafür konfiguriert ist, als Reaktion auf eine Operation, bei der ein Signal in den Sensor (2) eingegeben wird, eine Kommunikation durchzuführen,
ein Mundstück (61, 62, 63), das den Sensor (2) und die Kommunikationseinheit (3) hält und dafür konfiguriert ist, an einer Zahnkrone (T) eines Subjekts befestigt zu werden, und zumindest einen Teil eines Zahnfleischs (G) des Subjekts bedeckt,
ein Licht absorbierendes Element (215) und
eine Feststellungseinheit (8),
wobei der Sensor (2) ein Temperatursensor ist, der einen Temperaturdetektor (22) einschließt, der dafür konfiguriert ist, eine Temperatur zu erfassen,
die Kommunikationseinheit (3) dafür konfiguriert ist, die Kommunikation als Reaktion auf eine Operation durchzuführen, bei welcher der Temperaturdetektor (22) einen Temperaturanstieg, gleich einem vorbestimmen ersten Schwellenwert oder größer als derselbe, erfasst,
wobei das Mundstück (61, 62, 63) aus einem lichtdurchlässigen Material hergestellt ist und zumindest den Temperaturdetektor (22) in dem Sensor (2) bedeckt und
wobei die Feststellungseinheit (8) dafür konfiguriert ist, festzustellen, dass das Mundstück (61, 62, 63) an der Zahnkrone (T) des Subjekts befestigt ist, wenn der Sensor (2) eine Temperatur, gleich einem vorbestimmen zweiten Schwellenwert oder größer als derselbe, erfasst, und festzustellen, dass das Mundstück (61, 62, 63) nicht an der Zahnkrone (T) des Subjekts befestigt ist, wenn der Sensor (2) eine Temperatur, kleiner als der vorbestimme zweite Schwellenwert, erfasst,
**dadurch gekennzeichnet, dass**
das Licht absorbierende Element (215) in einem Bereich, der mit Licht bestrahlt wird, bereitgestellt wird, um das Licht zu absorbieren,
wobei die Kommunikationseinheit (3) dafür konfiguriert ist, die Kommunikation als Reaktion auf eine Operation durchzuführen, bei welcher der Temperaturdetektor (22) einen Temperaturanstieg, gleich einem vorbestimmen ersten Schwellenwert oder größer als derselbe, erfasst, wenn der Temperaturdetektor (22) mit dem Licht bestrahlt wird.

8. Intraorale biologische Überwachungseinrichtung (11) nach Anspruch 7,
wobei die Kommunikationseinheit (3) dafür konfiguriert ist, eine Temperatur von 60 Grad Celsius als den vorbestimmten ersten Schwellenwert anzuwenden.

9. Intraorale biologische Überwachungseinrichtung (11) nach Anspruch 7,
wobei die Kommunikationseinheit (3) dafür konfiguriert ist, eine Temperatur von 150 Grad Celsius oder höher als den vorbestimmten ersten Schwellenwert anzuwenden.

10. Intraorale biologische Überwachungseinrichtung (11) nach einem der Ansprüche 7 bis 9,
wobei der Sensor (2) intermittierend betrieben wird.

## Revendications

1. Dispositif de surveillance biologique intra-orale (11) comprenant :
un capteur (2) ;
une unité de communication (3) qui est configurée pour mettre en œuvre une communication en réponse à une opération dans laquelle un signal est entré dans le capteur (2) ;
une pièce buccale (61, 62, 63) qui maintient le capteur (2) et l'unité de communication (3) et qui est configurée pour être fixée à une couronne (T) d'un sujet et recouvre au moins une partie de la gencive (G) du sujet ; et
une unité de détermination (8),
dans lequel le capteur (2) est un capteur optique comprenant un photodétecteur (21) qui est configuré pour détecter la lumière,
la pièce buccale (61, 62, 63) est constituée d'un matériau transmettant la lumière et recouvre au moins le photodétecteur (21) du capteur (2), et
l'unité de communication (3) est configurée pour mettre en œuvre la communication en réponse à une opération dans laquelle la lumière est détectée par le photodétecteur (21),
**caractérisé en ce que**
le photodétecteur (21) est configuré pour faire face à au moins un élément parmi la couronne (T) et la gencive (G) du sujet par fixation de la pièce buccale (61, 62, 63) à la couronne (T) du sujet, et
l'unité de détermination (8) est configurée pour déterminer que la pièce buccale (61, 62, 63) est fixée à la couronne (T) du sujet quand le capteur (2) ne détecte pas la lumière, et pour déterminer que la pièce buccale (61, 62, 63) n'est pas fixée à la couronne (T) du sujet quand le capteur (2) détecte la lumière.

2. Dispositif de surveillance biologique intra-orale (11) selon la revendication 1,
dans lequel le capteur (2) est disposé dans au moins une partie de la couronne (T) du sujet qui n'entre pas en contact avec d'autres dents, même quand le sujet occlut une dent d'une mâchoire supérieure et une dent d'un mâchoire inférieure ainsi que la gencive (G) du sujet.

3. Dispositif de surveillance biologique intra-orale (11) selon la revendication 1 ou 2,
dans lequel le photodétecteur (21) est configuré pour détecter la lumière ayant une longueur d'onde appartenant à la plage du proche infrarouge ou à la plage de la lumière visible.

4. Dispositif de surveillance biologique intra-orale (11) selon l'une quelconque des revendications 1 à 3,
dans lequel le photodétecteur (21) est configuré pour détecter la lumière soumise à une modulation prédéterminée.

5. Dispositif de surveillance biologique intra-orale (11) selon la revendication 1,
dans lequel le photodétecteur (21) comprend un élément photovoltaïque (211) qui est configuré pour recevoir la lumière afin de générer de l'énergie électrique, et pour détecter la lumière en utilisant l'énergie électrique générée par l'élément photovoltaïque (211).

6. Dispositif de surveillance biologique intra-orale (11) selon l'une quelconque des revendications 1 à 5,
dans lequel le capteur (2) fonctionne par intermittence.

7. Dispositif de surveillance biologique intra-orale (11) comprenant :
un capteur (2) ;
une unité de communication (3) qui est configurée pour mettre en œuvre une communication en réponse à une opération dans laquelle un signal est entré dans le capteur (2) ;
une pièce buccale (61, 62, 63) qui maintient le capteur (2) et l'unité de communication (3) et qui est configurée pour être fixée à une couronne (T) d'un sujet et recouvre au moins une partie de la gencive (G) du sujet ;
un élément absorbant la lumière (215), et
une unité de détermination (8),
dans lequel le capteur (2) est un capteur de température comprenant un détecteur de température (22) qui est configuré pour détecter une température,
l'unité de communication (3) est configurée pour mettre en œuvre la communication en réponse à une opération dans laquelle le détecteur de température (22) détecte une augmentation de température supérieure ou égale à une première valeur seuil prédéterminée,
dans lequel la pièce buccale (61, 62, 63) est constituée d'un matériau transmettant la lumière et
recouvre au moins le détecteur de température (22) dans le capteur (2), et
dans lequel l'unité de détermination (8) est configurée pour déterminer que la pièce buccale (61, 62, 63) est fixée à la couronne (T) du sujet quand le capteur (2) détecte une température supérieure ou
égale à une deuxième valeur seuil prédéterminée, et
pour déterminer que la pièce buccale (61, 62, 63) n'est pas fixée à la couronne (T) du sujet quand le capteur (2) détecte une température inférieure à la deuxième valeur seuil prédéterminée,
**caractérisé en ce que**
l'élément absorbant la lumière (215) est pourvu dans une région irradiée par la lumière pour absorber la lumière,
dans lequel l'unité de communication (3) est configurée pour mettre en œuvre la communication en réponse à une opération dans laquelle le détecteur de température (22) détecte une augmentation de température supérieure ou égale à la première valeur seuil prédéterminée quand le détecteur de température (22) est irradié par la lumière.

8. Dispositif de surveillance biologique intra-orale (11) selon la revendication 7,
dans lequel l'unité de communication (3) est configurée pour adopter une température de 60 degrés Celsius comme ladite première valeur seuil prédéterminée.

9. Dispositif de surveillance biologique intra-orale (11) selon la revendication 7,
dans lequel l'unité de communication (3) est configurée pour adopter une température supérieure ou égale à 150 degrés Celsius comme ladite première valeur seuil prédéterminée.

10. Dispositif de surveillance biologique intra-orale (11) selon l'une quelconque des revendications 7 à 9,
dans lequel le capteur (2) fonctionne par intermittence.
